# EUROPEAN PATENT SPECIFICATION

(11) **EP 0 923 576 B1**
(45) Date of publication and mention of the grant of the patent: **06.11.2002**
(21) Application number: 97938630.7
(22) Date of filing: 26.08.1997
(51) Int. Cl.: C07D 409/12, A61K 31/40

(54) **4-AMINOETHOXY-INDOLONE DERIVATIVES AS DOPAMINE D2 AGONISTS**
4-AMINOETHOXY-INDOLONDERIVATE ALS DOPAMIN D2 AGONISTEN
DERIVES DE 4-AMINOETHOXY-INDOLONE UTILISES EN TANT QU'AGONISTES DU RECEPTEUR D2 DE LA DOPAMINE

(30) Priority: 27.08.1996 US 703606
(43) Date of publication of application: 23.06.1999
(73) Proprietor: Wyeth, Madison, New Jersey 07940-0874 (US)
(72) Inventor: MEWSHAW, Richard Eric, Princeton, NJ 08540 (US); WEBB, Michael Byron, Levittown, PA 19054 (US)
(74) Representative: Mannion, Sally Kim, Dr.
(86) International application number: US9715027
(87) International publication number: WO98008843

(56) References cited:
- EP-A- 0 771 801
- US-A- 5 541 199

## Description

### BACKGROUND OF INVENTION

Efforts to induce antipsychotic activity with dopamine autoreceptor agonists have been successful (Dorsini et al. Adv. Biochem. Psychopharmacol 16, 645-648, 1977; Tamminga et al. Science 200, 567-568, 1975; and Tamminga et al. Psychiatry 398-402, 1986). A method for determining intrinsic activity at the dopamine D2 receptor was recently reported [Lahti et al., Mol. Pharm. 42, 432-438, (1993)]. Intrinsic activity is predicted using the ratio of the "low-affinity agonist" (LowAg) state of the receptor and the "high-affinity agonist" (HighAg) state of the receptor, i.e. LowAg/HighAg. These ratios correlate with the agonist, partial agonist, and antagonist activities of a given compound, which activities characterize a compounds ability to elicit an antipsychotic effect.

U.S. Pat. No. 4,314,944 to Huffman and Wilson describes a series of indolones which are useful for cardiovascular abnormalities. NL-009247 describes a series of 2-hydroxy-3-aminopropoxy) oxindoles which are useful as β-adrenergic blocking agents for the treatment of heart diseases and WO 9403425 discloses a series of heterocyclic derivatives useful in the treatment of cardiovascular diseases.

### DESCRIPTION OF THE INVENTION

In accordance with this invention, there is provided a group of compounds which have potency at the dopamine D₂ receptor. These compounds are depicted by the following Formula I:

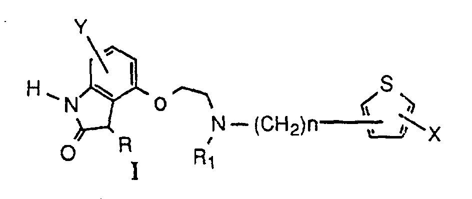

The compounds of this invention are useful antipsychotic agents essentially free from extrapyramidal side effects (EPS). The compounds of this invention are dopamine agonists exhibiting various degrees of intrinsic activity, some of which are selective autoreceptor agonists, and therefore partial agonist (i.e. activate only autoreceptors versus postsynaptic D₂ dopamine receptors). As such, they provide functional modulation of the dopamine systems of the brain without the excessive blockade of the postsynaptic dopamine receptors which have been observed to be responsible for the serious side effects frequently exhibited by agents found otherwise clinically effective for the treatment of schizophrenia. Activation of the dopamine autoreceptors results in reduced neuronal firing a well as inhibition of dopamine synthesis and release and therefore provide a means of controlling hyperactivity of the dopaminergic systems. The compounds of this invention were also found to have high intrinsic activity and therefore they can behave as the natural neurotransmitters i.e. as full agonists. As such, they are useful in the treatment of diseases having abnormal concentrations of dopamine in that they can be used as dopamine surrogates in the treatment of disease states such as Parkinson's disease.

More specifically, the compounds of this invention are 4-aminoethoxy-1,3-dihydro-indol-2-one derivatives which are illustrated by Formula I. 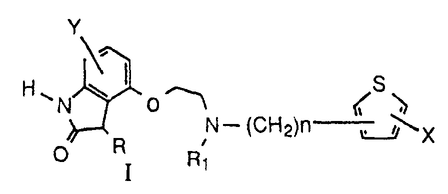 in which:
Y is hydrogen, halogen or alkoxy of 1 to 6 carbon atoms;
R is hydrogen or alkylthio of 1 to 6 carbon atoms;
R₁ is hydrogen or alkyl of 1 to 6 carbon atoms;
X is hydrogen, halogen, alkoxy of 1 to 6 carbon atoms, alkyl of 1 to 6 carbon atoms or phenyl;
n is one of the integers 1, 2, 3 or 4;
or a pharmaceutically acceptable salt thereof.

A preferred group of compounds are those in which Y is hydrogen, chloro or fluoro; R is hydrogen or alkylthio of 1 to 3 carbon atoms; R1 is hydrogen or alkyl of 1 to 3 atoms; X is hydrogen and n is one of the integers 2 and 3; or a pharmaceutically acceptable salt thereof.

The compounds of this Formula I also may be used in the form of a pharmaceutically acceptable acid addition salt having the utility of the free base. Such salts, prepared by methods well known to the art are formed with both inorganic or organic acids, for example: fumaric, maleic, benzoic, ascorbic, pamoic, succinic, bismethylenesalicylic, methanesulfonic, ethanedisulfonic, acetic, oxalic, propionic, tartaric, salicyclic, citric, gluconic, lactic, malic, mandelic, cinnamic, citraconic, aspartic, stearic, palmitic, itaconic, glycolic, p-aminobenzoic, glutamic, benzenesulfonic, hydrochloric hydrobromic, sulfuric, cyclohexylsulfamic, phosphoric and nitric acids.

The compounds of Formula I are generally prepared by the overall sequence indicated in Schemes I and II as follows:

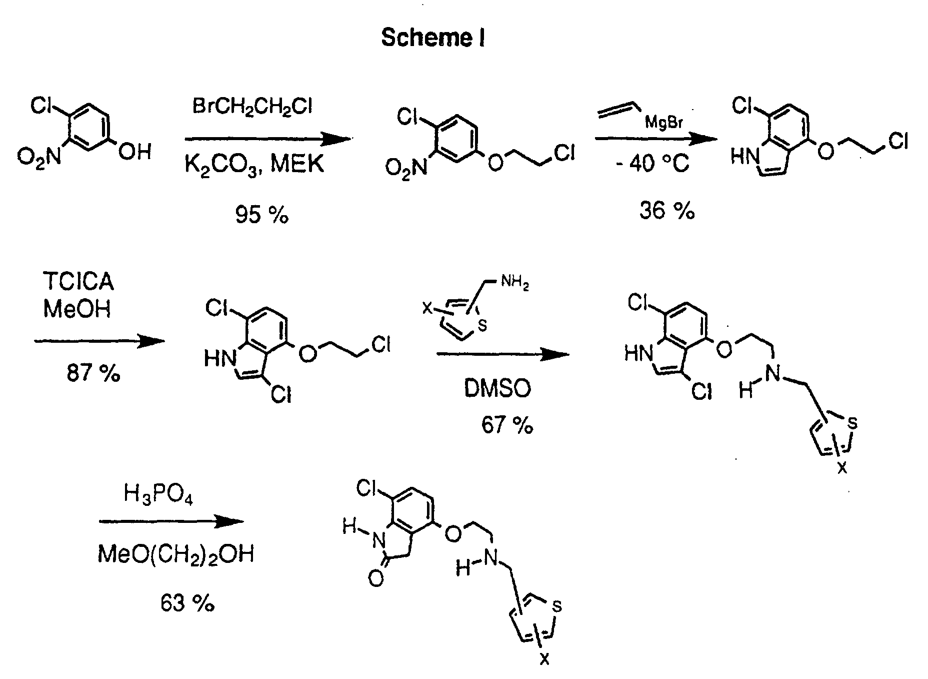

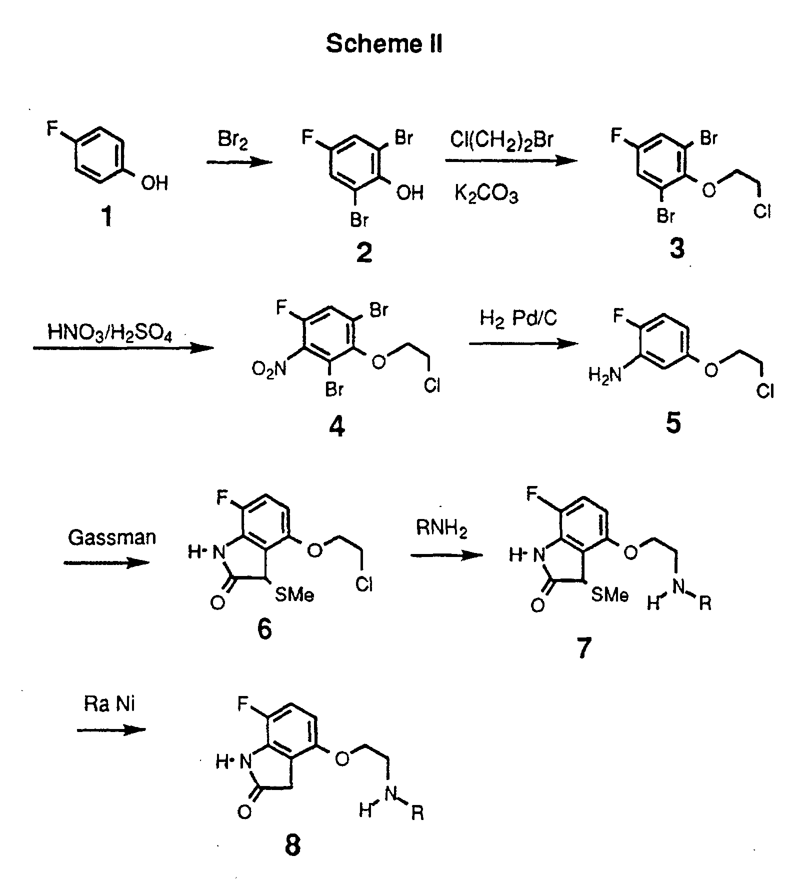

The following examples of the compounds of this invention and their production are presented by way of illustration.

### Intermediate 1

### 1-(2-Chloroethoxy)-4-chloro-3-nitrobenzene

### Method A

To a 2L 3-neck round-bottom flask was added 4-chloro-3-nitro-phenol (50 g, 0.29 mol), potassium carbonate (100g, 0.72 mol), dichloroethane (315 g, 3.2 mol), potassium iodide (5 g) and 2-butanone (1 L). The mixture was mechanically stirred and heated to reflux for 44 hours then allowed to cool to room temperature and the solids were filtered. The solvent was evaporated under vacuum and the oil dissolved in diethyl ether (300 mL) and washed with 10 % sodium hydroxide. The organic layer was dried over anhydrous magnesium sulfate , filtered, and the solvent removed under vacuum. The product was dissolved in 1:1 methylene chloride-hexanes and filtered through silica. Upon concentration 54.5 g (78.% %) of product was afforded as a white solid: mp 46-48 °C; MS EI m/e 235, 237, 239 (M⁺); ¹H NMR (400 MHz, DMSO-d₆) δ 3.95 (t, 2H, J=5.2 Hz), 4.36 (t, 2H, J=5.2 Hz), 7.32 (dd, 1H, J=3.2, J=8.9 Hz), 7.66, (d, 1H, J=9 Hz), 7.69, (d, 1H, J=3.2 Hz).

| Elemental analysis for C₈H₇Cl₂NO₃ | | | |
|---|---|---|---|
| Calcd | C, 40.71; | H, 2.99; | N, 5.93 |
| Found | C, 40.89, | H, 2.70; | N, 5.83 |

### Intermediate 1

### 1-(2-Chloroethoxy)-4-chloro-3-nitrobenzene

### Method B

To a 500 mL 3-neck round-bottom flask was added 4-chloro-3-nitro-phenol (10 g, 0.058 mol), potassium carbonate (20g, 0.14 mol), bromochloroethane (34.5 g, 0.24 mol) and 2-butanone (200 mL). The mixture was mechanically stirred and heated to reflux for 20 hours under nitrogen then allowed to cool to room temperature and the solids were filtered. The solvent was evaporated under vacuum and the oil dissolved in diethyl ether (300 mL) and washed with 10 % sodium hydroxide. The organic layer was dried over anhydrous magnesium sulfate, filtered and the solvent removed under vacuum. The product was dissolved in 1:1 methylene chloride-hexanes and filtered through a short pad silica. Upon concentration and standing 12.9 g (94.8.% %) of light yellow crystalline solid was afforded: mp 46-48 °C; MS EI m/e 235, 237, 239 (M⁺); ¹H NMR (400 MHz, DMSO-d₆) δ 3.95 (t, 2H, J=5.2 Hz), 4.36 (t, 2H, J=5.2 Hz), 7.32 (dd, 1H, J=3.2, J=8.9 Hz), 7.66, (d, 1H, J=9 Hz), 7.69, (d, 1H, J=3.2 Hz).

| Elemental analysis for C₈H₇Cl₂NO₃ | | | |
|---|---|---|---|
| Calcd | C, 40.71; | H, 2.99; | N, 5.93 |
| Found | C, 40.89, | H, 2.70; | N, 5.83 |

### Intermediate 2

### 7-Chloro-4-(2-chloroethoxy)-1H-indole

To a solution of 1-(2-chloroethoxy)-4-chloro-3-nitrobenzene (10.00 g, 0.04236 mol) in THF (230 mL) stirred in a cold bath at -50 to -40 °C was added a THF solution of vinylmagnesium bromide (132 mL, 1.0 M, 0.132 mol) over 2 minutes. After stirring in the cold bath for 2-2.5 hours, saturated NH₄Cl (150 mL) was added to the cold solution and it was removed from the cold bath. 1 M HCl was added to dissolve the precipitated solids and the mixture was stirred for 0.5 hour. The layers were separated and the aqueous phase was extracted once with diethyl ether (80 mL). The organic layer combined and dried over anhydrous magnesium sulfate, filtered and the solvent evaporated to give 15.43 g of a dark oil. Purification by chromatography (hexanes-methylene chloride, 2:1) afforded a solid which was triturated with hexane and filtered to afford the product as yellowish white solid: 3.51 g (36%); mp 73-75 °C; MS EI m/e 229, 231, 233 (M⁺);¹H NMR (400 MHz, DMSO-d₆) δ 3.99 (t, 2H;, J=5.1 Hz), 4.34 (t, 2H, J=5.0 Hz), 6.51 (t, 1H, J=2.7 Hz), 6.53 (d, 1H, J=7.8 Hz), 7.04 (d, 1H, J=8.0 Hz), 7.29 (t, 1H, J=2.7 Hz), 11.43 (s, 1H).

| Elemental analysis for C₁₀H₉Cl₂NO | | | |
|---|---|---|---|
| Calcd | C, 52.20; | H, 3.94; | N, 6.09 |
| Found | C, 52.09; | H, 3.92; | N, 5.96 |

### Intermediate 3

### Method A

### 3,7-Dichloro-4-(2-chloroethoxy)-1H-indole

To a solution of 7-chloro-4-(2-chloroethoxy)-1H-indole (4.61 g, 20.0 mmol) in acetonitrile (100 mL) was added N-chlorosuccimide (2.94 g, 2.20 mmol) at room temperature. the reaction was allowed to stir for 1.5 h then poured into water (100 mL) and extracted with methylene chloride (200 mL). The organic layer was dried over anhydrous magnesium sulfate, filtered, and the solvent removed under vacuum to afford a dark solid. This material was chromatographed (methylene chloride-hexanes: 1:2) to afford 4.15 g (78.4 %) as a white solid: mp 106-107.5 °C; IR (KBr) 3400 cm-1; MS EI m/e 263, 265, 267, 269 (M+); ¹H NMR (CDCl₃) δ 3.91 (2H, t, J=6.2 Hz), 4.33 (2H, t, J=6.2 Hz), 6.47 (1H, d, J= 8.4 Hz), 7.08-7.13 (2H, m), 8.26 (1H, bs, NH).

| Elemental analysis for C₂₂H₂₅N₂O₃Cl | | | |
|---|---|---|---|
| Calc'd | C, 45.40; | H, 3.05; | N, 5.30 |
| Found | C, 44.64; | H, 2.74; | N, 5.16 |

### Intermediate 3

### Method B

### 3,7-Dichloro-4-(2-chloroethoxy)-1H-indole

To a solution of 7-chloro-4-(2-chloroethoxy)-1H-indole (10.0 g, 37.8 mmol) in methanol (200 mL) under nitrogen containing sodium acetate (6.0 g) and acetic acid (1 mL) was added portionwise trichloroisocyanic acid (4.0 g, 17.2 mmol) at 2 °C. The reaction temperature was maintained below 8 °C was allowed to stir for 4 hours at which time it was diluted with diethyl ether (200 mL) and washed with 10 NaOH. The organic layer dried over anhydrous magnesium sulfate, filtered and the solvent removed under vacuum to afford an oil. This material was dissolved in methylene chloride and filtered over a short pad of silica and concentrated to afford 10.0 g (87.0%) a yellowish white solid: mp 106-107.5 °C; IR (KBr) 3400 cm-1; MS EI m/e 263, 265, 267, 269 (M+); ¹H NMR (CDCl₃) δ 3.91 (2H, t, J=6.2 Hz), 4.33 (2H, t, J=6.2 Hz), 6.47 (1H, d, J= 8.4 Hz), 7.08-7.13 (2H, m), 8.26 (1H, bs, NH).

### Intermediate 4

### N-Thien-2-ylmethyl-[2-(3,7-dichloro-1H-indol-4-yloxy-ethyl]-ethylamine

A solution of the 2-(3,7-dichloro-1H-indol-4-yloxy)-chloroethane (1.70 g, 6.43 mmol) and 2-thienylmethylamine (2.91 g, 25.7 mmol) in anhydrous dimethylsulfoxide (6 mL) was heated to 90 °C for 12 hours. The reaction mixture was poured into water (150 mL) and made basic with 0.1 N NaOH then extracted with methylene chloride (2x 100 mL). The organic layers were combined and dried over anhydrous magnesium sulfate, filtered, and the solvent concentrated. Purfication with flash chromatography (ethyl acetate-hexanes; 1:1) afforded 1.68 g (76.3%) of an orangish solid: mp 99-101°C.

| Elemental analysis for C₁₅H₁₅Cl₂N₂OS | | | |
|---|---|---|---|
| Calc'd | C, 52.79; | H, 4.13; | N, 8.21 |
| Found | C, 52.70; | H, 3.95; | N, 8.19 |

(4b) N-Thien-3-ylmethyl-[2-(3,7-dichloro-1H-indol-4-yloxy-ethyl]-ethylamine was prepared similarly by using 3-thienylmethylamine and isolated as a yellow solid: mp 123-125 °C; MS El m/e 340/342/344 (M+).

| Elemental analysis for C₁₈H₁₈Cl₂N₂O | | | |
|---|---|---|---|
| Calc'd | C, 52.79; | H, 4.14; | N, 8.21 |
| Found | C, 52.87; | H, 4.05 | N, 8.18 |

### Intermediate 5

### 2,6-Dibromo-4-fluoro-phenol

To a solution of 4-fluorophenol (25 g, 0..22. mol) in acetic acid (200 mL) at room temperature was slowly added dropwise bromine (78 g, 0.49 mol) while being mechanically stirred. After 1 hour the reaction mixture was poured into ice water (1.5 L) followed by 100 mL of saturated aqueous sodium bisulfite. The solid precipitate was filtered and dried to afford 51.8 g (86.0%) a white solid: mp 54-55 °C; ¹H NMR (CDCl₃) δ 5.69 (1H, s, OH), 7.25 (2H, d, J=7.5 Hz); MS EI m/e 268/270/272 (M⁺).

| Elemental analysis for C₆H₃Br₂FO | | |
|---|---|---|
| Calcd | C, 26.70; | H, 1.12 |
| Found | C, 26.64; | H, 1.07 |

### Intermediate 6

### 1-(2-Chloroethoxy)-2,6-dibromo-4-fluorobenzene

A mixture of 2,6-dibromo-4-fluoro-phenol (55 g, 0.20 mol), potassium carbonate (60 g, 0.43 mol), 1-bromo-2-chloroethane (32.5 g, 0.23 mol) and 2-butanone (500 mL) was heated to reflux for 2 hours and allowed to cool to ambient temperature. The solids were filtered and the solvent was removed under vacuum to afford an oil. The oil was dissolved in diethyl ether (300 mL) and washed with water, dried over anhydrous magnesium sulfate, charcaolized, and filtered through Solka floc to afford 65.9 g (97.2 %) of an oil; MS EI m/e 330/332/334/336 (M⁺); ¹H NMR (.CDCl₃) δ 3.89 (2H, t, J=6.1 Hz), 4.23 (2H, t, J=6.1 Hz), 7.28 (2H, d, J=7.5 Hz).

### Intermediate 7

### 1-(2-Chloroethoxy)-2,6-dibromo-4-fluoro-3-nitrobenzene

To a solution of 1-(2-chloroethoxy)-2,6-dibromo-4-fluorobenzene (65.8 g, 0.20 mol) in concentrated sulfuric acid (165 mL) maintained at room temperature using a water bath was slowly added a solution of nitric acid in sulfuric acid (10 mL HNO₃ in 165 mL H₂SO₄). The reaction was allowed to stir at room temperature for 1 hour then poured into ice (1.5 L) and extracted with methylene chloride (2x 300 mL). The combined organic layers were washed with aqueous sodium bicarbonate (150 mL) and dried over anhydrous magnesium sullfate, filtered, and the solvent removed under vacuum to afford 73.3 g (97.1 %) a white crystalline solid: mp 56-57 °C; MS EI m/e 375/377/379/381; ¹H NMR (CDCl₃) δ 3.91 (2H, t, J=5.9 Hz), 4.29 (2H, d, J=5.9 Hz), 7.54 (8.1 Hz).

| Elemental analysis for C₈H₅Br₂ClFNO₃ | | | |
|---|---|---|---|
| Calcd | C, 25.46; | H, 1.34; | N, 3.71 |
| Found | C, 25.46; | H, 1.20; | N, 3.51 |

### Intermediate 8

### 1-(2-Chloroethoxy)-4-fluoro-3-aminobenzene

A solution of 1-(2-chloroethoxy)-2,6-dibromo-4-fluoro-3-nitrobenzene (73.2 g, 0.19 mol) in ethanol (1.1 L) containing 7.3 g of 10% palladium on carbon was hydrogenated at 40 psi for 5 days. The catalyst was filtered and the solvent was removed. The residue was dissolved in diethyl ether (300 mL) and washed with saturated aqueous sodium carbonate (200 mL). The organic layer separated and washed with water, dried over anhydrous magnesium sulfate, filtered, and the solvent removed to afford an oil which solidies to afford 32.5 g (90.0 %) a dark solid: mp 42-43 °C; MS EI m/e 189/191 (M⁺); 1H NMR (CDCl₃) δ 3.40-3.60 (2H, bs, NH₂), 3.77 (2H, d, J=6 Hz), 4.14 (2H, d, J=6 Hz), 6.19-6.23 (1 H, m), 6.36 (1H, dd, J=7, 3 Hz), 6.88 (1H, dd, J=11, 9 Hz).

| Elemental analysis for C₈H₉ClFNO | | | |
|---|---|---|---|
| Calcd | C, 50.68; | H, 4.78; | N, 7.39 |
| Found | C, 50.46; | H, 4.66; | N, 7.46 |

### Intermediate 9

### 4-(2-Chloroethoxy)-7-fluoro-3-thiomethyl-1,3-dihydro-indol-2-one

To a solution of ethyl(methylthio)acetate (7.2 g, 53.4 mmol) in anhydrous methylene chloride (200 mL) at -78 °C was added sulfuryl chloride (8.1 g, 59.7 mmol) and stirred for 20 minutes. A solution of 1-(2-chloroethoxy)-4-fluoro-3-aminobenzene (10.0 g, 52.8 mmol) and Proton Sponge (13.9 g) in methylene chloride (100 mL) was added dropwise and stirred for 2 hours, followed by the addition of triethylamine (6.5 g, 64.5 mmol). The temperature was maintained at -78 °C and the reation mixture was allowed to stir for 1 hour. After warming to room temperature, the mixture was poured into brine (200 mL) and dried over anhydrous magnesium sulfate, filtered, and the solvent removed to afford an oil. Acetic acid (75 mL) was added to the oil and the mixture allowed to stand for 18 hours then the solvent was removed under vacuum. The residue was partitioned between diethyl ether (400 mL) and 2.5 N aqueous hydrochloric acid (150 mL). The organic layer was separated and dried over anhydrous magnesium sulfate, filtered, and the solvent removed to afford a solid. Trituration of the solid with a small amount of diethyl ether (30 mL) afforded 8.8 g (60.5 %) a yellow solid: mp 140-141 °C; MS El m/e 275/277 (M⁺); ¹NMR (CDCl₃) δ 2.14 (3H, s), 3.79-3.87 (2H, m), 4.25-4.33 (2H, m), 4.35 (1H, s), 6.51 (1H, dd, J=9.1, 3.3 Hz), 6.99 (1H, app. t, J=9.1 Hz), 8.09 (1H, s).

| Elemental analysis for C₁₁H₁₁ClFNO₂S | | | |
|---|---|---|---|
| Calcd | C, 47.92; | H, 4.02; | N, 5.08 |
| Found | C, 47.67; | H, 3.85; | N, 4.85 |

### EXAMPLE I

### 4-[2-(Thien-3-ylmethyl)aminoethoxyl-7-fluoro-3-methylthio-1,3-dihydroindol-2-one

The title compound was prepared from 4-(2-chloroethoxy)-7-fluoro-3-thiomethyl-1,3-dihydro-indol-2-one (3.0 g, 10.9 mmol) and 3-thienylmethylamine (4.37 g, 38.6 mmol) in dimethylsulfoxide (50 mL) according to the procedure used to prepare intermediate 6 to afford 2.11 g (55 %) of a tan oil: MS El m/e 352 (M⁺). The fumarate salt was prepared in ethanol to afford a white solid: mp 152.5-155 C; ¹H NMR (CDCl₃) δ 1.93 (3H, s), 2.99 (2H, m), 3.93 (1 H, s), 4.11-4.20 (2H, m), 4.52 (1H, s), 6.55 (2H, s), 6.62 (1H, d, J=9., 3 Hz), 7.08-7.14 (2H, m), 7.41 (1H, m), 7.50 (1H, dd, J=5. 3 Hz), 11.00 (1H, bs); IR (KBr) 1710 cm-1.

| Elemental analysis for C₁₆H₁₇N₂FN₂O₂S₂•C₄H₄O₄ | | | |
|---|---|---|---|
| Calc'd | C, 51.27; | H, 4.52; | N, 5.98 |
| Found | C, 51.29; | H, 4.52; | N, 5.91 |

### EXAMPLE 2

### 4-[2-Thien-2-ylmethyl)aminoethoxyl-7-chloro-1,3-dihydro-indol-2-one

To a solution of N-2-thienyl-[2-(3,7-dichloro-1H-Indol-4-yloxy)-ethyl]-ethylamine (1.41 g, 4.12 mmol) in 2-methoxyethanol (17 mL) was added phosphoric acid(4 mL). The solution was refluxed for 4 hours then poured into H₂O (100 mL) and basified with 2.5 N NaOH until pH 9. The aqueous layer was extracted with methylene chloride (2x200 mL). Combination of the organic layers, drying over MgSO₄ and evaporation of the solvent gave 1.15 g (86.5%) of product; mp 154-155 °C.

To a hot solution of fumaric acid (6.6 mmol)) in EtOH (20 mL) was added a hot solution of the product (1.08 g, 3.3 mmol) in EtOH (50 mL). This mixture was cooled, filtered and dried to give hemi-fumarate salt of the title compound as a slightly yellow white powder: 0.5429 g (60%); mp 203-204 °C; MS EI m/e 323/324 (M⁺).

| Elemental analysis for C₁₅H₁₅ClN₂SO₂•0.5C₄H₄O₄ | | | |
|---|---|---|---|
| Calcd | C, 53.61; | H, 4.50; | N, 7.36 |
| Found | C, 53.41; | H, 4.39; | N, 7.17 |

(2b) 4-[2-(Thien-3-ylmethyl)aminoethoxy]-7-chloro 1,3-dihydro-indol-2-one was prepared in the manner of the preceding Example 2 (40 % yield). The fumarate salt was prepared from ethanol as a light brown solid: mp 191.5-193 °C; MS EI m/e 323/324 (M⁺).

| Elemental analysis for C₁₅H₁₅ClN₂SO₂•0.5C₄H₄O₄ | | | |
|---|---|---|---|
| Calcd | C, 53.61; | H, 4.50; | N, 7.36 |
| Found | C, 53.27; | H, 4.51; | N, 7.17 |

### EXAMPLE 3

### 4-[2-(Thien-3-ylmethyl)aminoethoxyl-7-fluoro-1,3-dihydro-indol-2-one

To a solution of 4-[(2-(3-thienyl)-(aminoethoxy)]-7-fluoro-3-thiomethyl-1,3-dihydro-indol-2-one (1.80 g, 5.12 mmol) in ethanol (30 mL) was added intermittently an excess of Raney nickel at room temperature. After 5 hours the complete disappearance of starting material was observed and the catalyst was filtered and the solvent removed and the solid dissolved in a minimum amount of a solution of methanol in methylene chloride and passed through a silica gel column (5% methanol in methylene chloride) to afford 330 mg (21 %) a light yellow solid; mp 154-157 °C; ¹H NMR (DMSO-d₆) δ 2.80 (2H, t, J=6 Hz), 3.42 (2H, s), 3.74 (2H, s), 4.03 (2H, t, J=6 Hz), 6.54 (1H, d, J=9, 3 Hz), 7.01 (1H, app t, J=9 Hz)7.18-7.34 (5H, m), 10.81 (1H, d, J=10 Hz). The fumarate salt was prepared in ethanol to afford a yellow solid: mp 186.5-187 °C.

| Elemental analysis for C₁₅H₁₅FN₂O₂S•0.5 C₂H₄O₄ | | | |
|---|---|---|---|
| Calc'd | C, 56.03; | H, 4.70; | N, 7.69 |
| Found | C, 55.81; | H, 4.74; | N, 7.34 |

(3b)4-[2-(Thien-2-ylmethyl)aminoethoxy]-7-fluoro-1,3-dihydro-indol-2-one was prepared in the same manner as the product of Example 3. The fumarate salt was prepared from ethanol as yellow crystals: mp 184-185.5 °C.

| Elemental analysis for C₁₅H₁₅FN₂O₂S•0.5 C₂H₄O₄ | | | |
|---|---|---|---|
| Calc'd | C, 56.03; | H, 4.70; | N, 7.69 |
| Found | C, 55.48; | H, 4.73; | N, 7.48 |

Affinity for the dopamine autoreceptor was established by a modification of the standard experimental test procedure of Seemen and Schaus, European Journal of Pharmacology 203, 105-109 (1991), wherein homogenized rat striatal brain tissue is incubated with ³H-quinpirole (Quin.)and various concentrations of test compound, filtered and washed and counted in a Betaplate scintillation counter.

High affinity for the dopamine D-2 receptor was established by the standard experimental test procedure of Fields, et al., Brain Res., 136, 578 (1977) and Yamamura et al., eds., Neurotransmitter Receptor Binding, Raven Press, N.Y. (1978) wherein homogenized limbic brain tissue is incubated with ³H-spiroperidol (Spiper.) and various concentrations of test compound, filtered and washed and shaken with Hydrofluor scintillation cocktail (National Diagnostics) and counted in a Packard 460 CD scintillation counter.

The results of these studies were as follows:

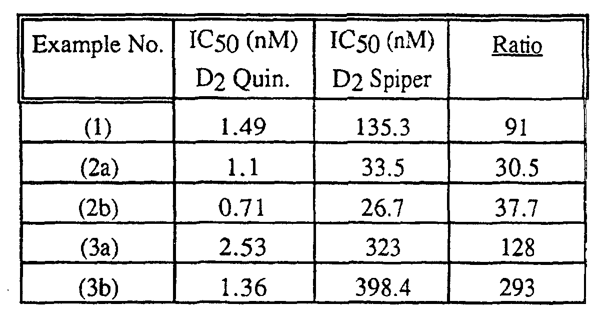

Hence, the compounds of this invention effect the synthesis of the neurotransmitter dopamine and thus are useful in the treatment of dopaminergic disorders such as schizophrenia, Parkinson's disease, Tourette's Syndrome, alcohol addiction, cocaine addiction, and addiction to analagous drugs.

Applicable solid carriers for pharmaceutical compositions containing the compounds of this invention can include one or more substances which may also act as flavoring agents, lubricants, solubilizers, suspending agents, fillers, glidants, compression aids, binders or tablet-disintergrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form.

The dosage to be used in the treatment of a specific psychosis must be subjectively determined by the attending physician. The variables involved include the specific psychosis and the size, age and response pattern of the patient.

## Claims

1. A compound of formula I 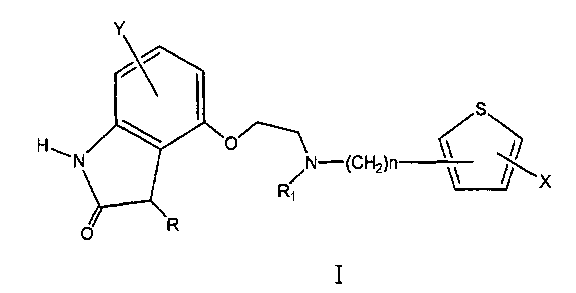 in which:
Y is hydrogen, halogen or alkoxy of 1 to 6 carbon atoms;
R is hydrogen or alkylthio of 1 to 6 carbon atoms;
R₁ is hydrogen or alkyl of 1 to 6 carbon atoms;
X is hydrogen, halogen, alkoxy of 1 to 6 carbon atoms, alkyl of 1 to 6 carbon atoms or phenyl;
n is one of the integers 1, 2, 3 or 4;
or a pharmaceutically acceptable salt thereof.

2. A compound of Claim 1 in which Y is hydrogen, chloro, fluoro, R is hydrogen or alkylthio of 1 to 3 carbon atoms; R₁ is hydrogen or alkyl of 1 to 3 atoms; X is hydrogen and n is one of the integers 2 and 3; or a pharmaceutically acceptable salt thereof.

3. The compound of Claim 1 which is 4-[2-(thien-3-ylmethyl)aminoethoxy]-7-fluoro-3-methylthio-1,3-dihydro-indol-2-one or a pharmaceutically acceptable salt thereof.

4. The compound of Claim 1 which is 4-[2-(thien-2-ylmethyl)aminoethoxy]-7-chloro-1,3-dihydro-indol-2-one or a pharmaceutically acceptable salt thereof.

5. The compound of Claim 1 which is 4-[2-(thien-3-ylmethyl)aminoethoxy]-7-chloro-1,3-dihydro-indol-2-one or a pharmaceutically acceptable salt thereof.

6. The compound of Claim 1 which is 4-[2-(thien-3-ylmethyl)aminoethoxyl-7-fluoro-1,3-dihydro-indol-2-one or a pharmaceutically acceptable salt thereof.

7. The compound of Claim 1 which is 4-[2-(thien-2-ylmethyl)aminoethoxy]-7-fluoro-1,3-dihydro-indol-2-one or a pharmaceutically acceptable salt thereof.

8. A pharmaceutical composition of matter comprising a compound of formula I as defined in any of claims 1 to 7, and a pharmaceutically acceptable carrier therefor.

9. A compound of formula I as defined in any of claims 1 to 7, for use in therapy.

10. Use of a compound of formula I as defined in any of claims 1 to 7 in the preparation of a medicament for reducing dopamine synthesis and release in a patient suffering from hyperactivity of the dopaminergic systems.

11. Use of a compound of formula I as defined in any of claims 1 to 7 in the preparation of a medicament for treatment of schizophrenia.

## Patentansprüche

1. Verbindung der Formel I 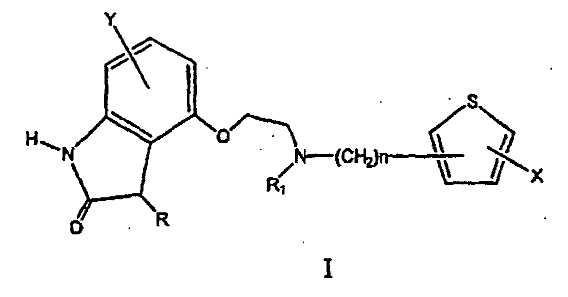 worin:
Y Wasserstoff, Halogen oder Alkoxy mit 1 bis 6 Kohlenstoffatomen darstellt;
R Wasserstoff oder Alkylthio mit 1 bis 6 Kohlenstoffatomen darstellt;
R₁ Wasserstoff oder Alkyl mit 1 bis 6 Kohlenstoffatomen darstellt;
X Wasserstoff, Halogen, Alkoxy mit 1 bis 6 Kohlenstoffatomen, Alkyl mit 1 bis 6 Kohlenstoffatomen oder Phenyl darstellt;
n eine der ganzen Zahlen 1, 2, 3 oder 4 ist;
oder ein pharmazeutisch annehmbares Salz davon.

2. Verbindung nach Anspruch 1, worin Y Wasserstoff, Chlor, Fluor darstellt, R Wasserstoff oder Alkylthio mit 1 bis 3 Kohlenstoffatomen darstellt; R₁ Wasserstoff oder Alkyl mit 1 bis 3 Atomen darstellt; X Wasserstoff darstellt und n eine der ganzen Zahlen 2 und 3 ist; oder ein pharmazeutisch annehmbares Salz davon.

3. Verbindung nach Anspruch 1, welche 4-[2-(Thien-3-ylmethyl)aminoethoxy)-7-fluor-3-methylthio-1,3-dihydro-indol-2-on oder ein pharmazeutisch annehmbares Salz davon ist.

4. Verbindung nach Anspruch 1, welche 4-[2-(Thien-2-ylmethyl)aminoethoxy]-7-chlor-1,3-dihydro-indol-2-on oder ein pharmazeutisch annehmbares Salz davon ist.

5. Verbindung nach Anspruch 1, welche 4-[2-(Thien-3-ylmethyl)aminoethoxy]-7-chlor-1,3-dihydro-indol-2-on oder ein pharmazeutisch annehmbares Salz davon ist.

6. Verbindung nach Anspruch 1, welche 4-[2-(Thien-3-ylmethyl)aminoethoxy]-7-fluor-1,3-dihydro-indol-2-on oder ein pharmazeutisch annehmbares Salz davon ist.

7. Verbindung nach Anspruch 1, welche 4-[2-(Thien-2-ylmethyl)aminoethoxy]-7-fluor-1,3-dihydro-indol-2-on oder ein pharmazeutisch annehmbares Salz davon ist.

8. Pharmazeutische chemische Zusammensetzung, welche eine Verbindung der Formel I, wie in einem der Ansprüche 1 bis 7 definiert, und einen pharmazeutisch annehmbaren Träger dafür umfasst.

9. Verbindung der Formel I, wie in einem der Ansprüche 1 bis 7 definiert, zur Verwendung in Therapie.

10. Verwendung einer Verbindung der Formel I, wie in einem der Ansprüche 1 bis 7 definiert, bei der Herstellung eines Arzneimittels zum Reduzieren von Dopamin-Synthese und Abgabe in einem Patienten, welcher an Hyperaktivität des dopaminergen Systems leidet.

11. Verwendung einer Verbindung der Formel I, wie in einem der Ansprüche 1 bis 7 definiert, bei der Herstellung eines Arzneimittels zur Behandlung von Schizophrenie.

## Revendications

1. Composé de formule I : 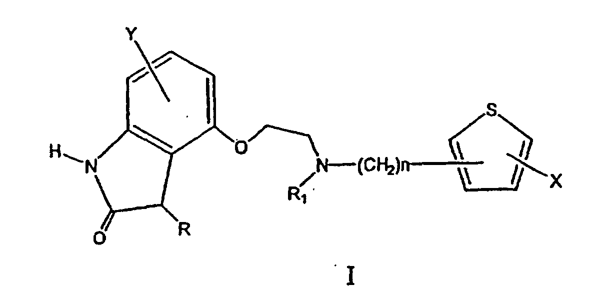 dans lequel :
Y est de l'hydrogène, un halogène ou un alcoxy de 1 à 6 atomes de carbone;
R est de l'hydrogène ou un alkylthio de 1 à 6 atomes de carbone;
R₁ est de l'hydrogène ou un alkyle de 1 à 6 atomes de carbone;
X est de l'hydrogène, un halogène, un alcoxy de 1 à 6 atomes de carbone, un alkyle de 1 à 6 atomes de carbone ou un phényle;
n est un des nombres entiers 1, 2, 3 ou 4;
ou un sel pharmaceutiquement acceptable de celui-ci.

2. Composé selon la revendication 1, dans lequel Y est de l'hydrogène, un chloro ou un fluoro, R est de l'hydrogène ou un alkylthio de 1 à 3 atomes de carbone; R₁ est de l'hydrogène ou un alkyle de 1 à 3 atomes de carbone; X est de l'hydrogène et n est un des nombres entiers 2 et 3; ou un sel pharmaceutiquement acceptable de celui-ci.

3. Composé selon la revendication 1, qui est le 4-[2-(thién-3-ylméthyl)aminoéthoxy]-7-fluoro-3-méthylthio-1,3-dihydro-indol-2-one ou un sel pharmaceutiquement acceptable de celui-ci.

4. Composé selon la revendication 1, qui est le 4-[2-(thién-2-ylméthyl)aminoéthoxy]-7-chloro-1,3-dihydro-indol-2-one ou un sel pharmaceutiquement acceptable de celui-ci.

5. Composé selon la revendication 1, qui est le 4-[2-(thién-3-ylméthyl)aminoéthoxy]-7-chloro-1,3-dihydro-indol-2-one ou un sel pharmaceutiquement acceptable de celui-ci.

6. Composé selon la revendication 1, qui est le 4-[2-(thién-3-ylméthyl)aminoéthoxy]-7-fluoro-1,3-dihydro-indol-2-one ou un sel pharmaceutiquement acceptable de celui-ci.

7. Composé selon la revendication 1, qui est le 4-[2-(thién-2-ylméthyl)aminoéthoxy]-7-fluoro-1,3-dihydro-indol-2-one ou un sel pharmaceutiquement acceptable de celui-ci.

8. Composition pharmaceutique comprenant un composé de formule I comme défini dans l'une quelconque des revendications 1 à 7, et un véhicule pharmaceutiquement acceptable prévu à cet effet.

9. Composé de formule I tel que défini dans l'une quelconque des revendications 1 à 7 pour utilisation en thérapie.

10. Utilisation d'un composé de formule I tel que défini dans l'une quelconque des revendications 1 à 7 dans la préparation d'un médicament pour réduire la synthèse et la libération de la dopamine chez un patient souffrant d'hyperactivité des systèmes dopaminergiques.

11. Utilisation d'un composé de formule I tel que défini dans l'une quelconque des revendications 1 à 7 dans la préparation d'un médicament pour le traitement de la schizophrénie.
